# EUROPÄISCHE PATENTSCHRIFT

(11) **EP 2 454 999 B1**
(45) Veröffentlichungstag und Bekanntmachung des Hinweises auf die Patenterteilung: **27.01.2016**
(21) Anmeldenummer: 10191535.3
(22) Anmeldetag: 17.11.2010
(51) Int. Cl.: A61B 10/00

(54) **Aufnahmehilfe für Stuhlproben mit abreißbaren Befestigungsbereichen**
Receiving device for stool samples with detachable attached sections
Aide à la réception pour coproculture dotée de zones de fixation déchirables

(43) Veröffentlichungstag der Anmeldung: 23.05.2012
(73) Patentinhaber: Rathenberg, Jürgen, 37581 Bad Gandersheim (DE)
(72) Erfinder: Rathenberg, Jürgen, 37581 Bad Gandersheim (DE)
(74) Vertreter: Hofstetter, Schurack & Partner

(56) Entgegenhaltungen:
- EP-A1- 0 860 371
- WO-A1-2004/075757
- DE-A1- 3 905 572
- DE-A1- 10 052 879
- DE-U1- 20 303 129
- DE-U1- 29 613 596

## Beschreibung

Die vorliegende Erfindung betrifft eine Aufnahmehilfe für WC-Toilettenbecken zur Aufnahme von Stuhlproben mit einem Aufnahmeblatt, das langgestreckt ist und dessen Länge ein Vielfaches seiner Breite beträgt, wobei an dem Aufnahmeblatt ein erster und mindestens zweiter Befestigungsbereich zur lösbaren Befestigung im Bereich eines Beckenrands eines WC-Toilettenbeckens vorgesehen ist.

Bei medizinischen Untersuchungen, insbesondere bei der Darmkrebsvorsorge und bei Stoffwechsel- sowie infektiösen Magen- Darmerkrankungen, ist es heute erforderlich, Stuhlproben zu untersuchen, die der Patient im häuslichen Bereich bei der Benutzung des Toilettenbeckens entnehmen muss. Diese Stuhlproben müssen für ein einwandfreies Untersuchungsergebnis unter hygienischen Bedingungen entnommen werden können.

Es hat sich gezeigt, dass es für ein einwandfreies Untersuchungsergebnis insbesondere erforderlich ist, einen Kontakt sowohl zwischen Stuhlprobe und Spülwasser als auch zwischen Stuhlprobe und Urin möglichst zu vermeiden. Dies bereitet jedoch bei modernen Sanitäranlagen, insbesondere bei so genannten Tiefspülbecken, Schwierigkeiten, da ein Kontakt zwischen Spülwasser, Urin und Stuhlprobe nicht vermieden wird. Ein solcher Kontakt verfälscht jedoch das Untersuchungsergebnis oder macht die Stuhlprobe für die Untersuchung unbrauchbar. Der Benutzer bzw. Patient hat also größte Schwierigkeiten, unter hygienischen Bedingungen eine Stuhlprobe zu entnehmen.

Aus der Patentschrift EP 1 596 721 B1 ist eine gattungsgemäße Aufnahmehilfe für WC-Toilettenbecken bekannt. Dementsprechend ist eine Aufnahmehilfe mit einem langgestreckten Aufnahmeblatt vorgesehen, bei dem durch das Aufnahmeblatt im Gebrauchszustand ein eine Mulde beschreibender Proben-Aufnahmebereich zur Verfügung gestellt wird. Durch die Ausbildung eines solchen muldenförmigen Stuhlprobenaufnahmebereichs kann sichergestellt werden, dass nicht nur fester Stuhlgang, sondern auch infektiöser, dünnflüssiger Stuhlgang nicht von dem Aufnahmeblatt herunterfließt und dadurch - in dem muldenartigen Stuhlproben-Aufnahmebereich gesammelt - für die Entnahme einer Stuhlprobe ohne vorherigen Kontakt mit Spülwasser oder Urin zur Verfügung steht. Die Aufnahmehilfe mit ihrem muldenartigen Aufnahmebereich stellt im Gebrauchszustand eine räumliche Struktur dar, die gleichwohl in der Herstellung, Lagerung und im Versand flach ist und sich vor der Verwendung nur in der Ebene erstreckt. Der eine Mulde beschreibende Stuhlproben-Aufnahmebereich wird durch eine Verwerfung des Aufnahmeblatts gebildet. Das Aufnahmeblatt ist langgestreckt und seine Länge beträgt ein Mehrfaches der Breite. Die Mulde weist Ränder auf, die durch die Längskanten des Aufnahmeblatts gebildet sind. Die Verwerfung des Aufnahmeblatts ist durch eine Verkürzung der wirksamen Länge der Längskanten des Aufnahmeblatts hervorgerufen. Die Verkürzung der wirksamen Länge der Längskanten des Aufnahmeblatts ist durch eine Faltung und eine belastungsfähige Formfixierung der Faltung eines ersten Bereichs am Seitenrand des Aufnahmeblatts und mindestens eines, dem ersten Bereich bezüglich der Längsachse des Aufnahmeblatt gegenüberliegenden, zweiten Bereichs am Seitenrand des Aufnahmeblatts ausgebildet. Die Aufnahmehilfe kann insbesondere Befestigungsbereiche aufweisen, auf die ein Klebstoff aufgetragen ist.

Als Klebstoffe für die Befestigungsbereiche kommen Polymerklebstoffe und beispielsweise Klebstoffe auf der Basis von Natur-Kautschuk in Frage. In der Regel sind aber auch die Klebstoffe auf der Basis des Natur-Kautschuks teilsynthetisch hergestellt und somit oft nicht vollständig oder nur sehr langsam biologisch abbaubar. Daher sind derartige Klebstoffe im Sinne einer umweltfreundlichen Entsorgung nicht über die Toilettenspülung, sondern über den üblichen Hausmüll zu entsorgen. Im kommunalen Abwasser bedingen nämlich unzulässig über die Toilette in den Abwasserpfad eingebrachte Abfälle erhebliche konstruktive Vorkehrungen und betrieblichen Aufwand, da verhindert werden muss, dass solches Material in die Gewässer gelangt oder in die biologischen Behandlungsstufen und die Anlagen zur Klärschlammstabilisierung auf den Kläranlagen eindringt. Hierzu werden an Mischwasserentlastungen und vor Kläranlagen mechanische Reinigungsstufen (Rechen, Siebe) betrieben, die durch Planung, Anschaffung und Betrieb sowie durch die Behandlung und Beseitigung der in ihnen anfallenden Abfälle erhebliche Kostenwirkung entfalten. Allein die Kosten für die Behandlung und Beseitigung des Rechenguts sind erheblich und liegen deutlich über denen einer vergleichbaren Entsorgung mit dem Hausmüll.

Darüber hinaus besteht das Problem, dass Müll in Kanalsystemen den Abfluss behindert. Unlösliche Stoffe sinken nämlich im Kanalsystem ab und stellen nicht nur in Trockenperioden ein großes Problem dar. Je mehr Müll in das Kanalsystem gelangt, desto höher sind die Wassermengen zu veranschlagen, die notwendig sind, um das Kanalsystem freizuhalten.

Aus der Druckschrift DE 100 52 879 A1 ist eine Aufnahmehilfe mit konvex und konkav gekrümmten Rändern bekannt. Die Befestigung der Aufnahmehilfe auf einer WC-Brille erfolgt mittels Klebestreifen auf Schlitzöffnungen der Aufnahmehilfe.

Weiterhin offenbart das Dokument DE 39 05 572 A1 eine Endlos-Papiermanschette zum Auffangen des Stuhls. Zum Auftrennen weist das endlose Band Perforationsnähte auf.

Das weitere Dokument DE 296 13 596 U1 zeigt eine Aufnahmehilfe, die einen muldenförmigen Einsatz besitzt. Dieser ist mit vier Bändern, welche jeweils Klebeschichten aufweisen, an einem WC-Sitz befestigbar.

Die Aufgabe der vorliegenden Erfindung besteht somit darin, eine Aufnahmehilfe für WC-Toilettenbecken zur Aufnahme von Stuhlproben bereitzustellen, die umweltgerechter entsorgt werden kann.

Erfindungsgemäß wird diese Aufgabe gelöst durch eine Aufnahmehilfe nach Anspruch 1.

In vorteilhafter Weise ist es somit möglich, mittels eines einfachen Handgriffs die Befestigungsbereiche vom übrigen Aufnahmeblatt zu trennen. Gewährleistet wird dieses Trennen durch ein Reißelement, das durch einfache manuelle Betätigung ein definiertes Abreißen der jeweiligen Befestigungsbereiche an vorgegebenen Stellen ermöglicht. Damit lässt sich das Aufnahmeblatt ohne die abgetrennten Befestigungsbereiche hygienisch in dem WC-Toilettenbecken entsorgen, während die Befestigungsbereiche, die in der Regel weniger gut biologisch abbaubar sind, beispielsweise über den Hausmüll entsorgt werden können.

Das Aufnahmeblatt ist rechteckförmig ausgebildet, und an jeder Breitseite ist jeweils einer der Befestigungsbereiche angeordnet. Eine derartige rechteckförmige Aufnahmehilfe ist einfach auf automatischem Wege herstellbar.

Dabei verläuft parallel zu jeder Breitseite jeweils das Reißelement zum Abreißen des jeweiligen Befestigungsbereichs. Damit definiert jedes Reißelement einen ebenfalls rechteckförmigen Befestigungsbereich, wodurch wiederum ein Herstellungsverfahren bereitgestellt werden kann, das sich durch unkomplizierte Verfahrensschritte auszeichnet.

Entsprechend einer besonders bevorzugten Ausführungsform ist das Aufnahmeblatt einteilig mit den Befestigungsbereichen gebildet. Beispielsweise besteht das Aufnahmeblatt zusammen mit seinen Befestigungsbereichen im Wesentlichen aus einem in Wasser langsam zerfallenden Material, insbesondere aus Papier. In dieses Aufnahmeblatt ist dann das Reißelement zu integrieren.

Jeder Befestigungsbereich weist bei einer bevorzugten Ausführungsform einen Klebstoff auf, der das lösbare Befestigen des Befestigungsbereichs an dem Beckenrand des WC-Toilettenbeckens gewährleistet. Insbesondere kann der Klebstoff ein aufgesprühter Schmelzhaftkleber sein. Der Klebstoff hat den Vorteil, dass mit ihm der Befestigungsbereich auf in der Regel glatten Toilettensitzen ohne die genaue Geometrie des Toilettensitzes berücksichtigen zu müssen, befestigt werden kann. Dabei erweist es sich als besonders vorteilhaft, wenn der Kleber aufgesprüht werden kann. Somit lässt sich die Herstellung in einer Fertigungsstraße mit einfachen Mitteln rasch realisieren.

Gemäß einer weiteren Ausführungsform weist jedes Reißelement zwei parallele Perforationslinien in dem Aufnahmeblatt auf, und ein Streifen des Aufnahmeblatts zwischen den parallelen Perforationslinien ist zum Abreißen des jeweiligen Befestigungsbereichs vom übrigen Aufnahmeblatt entlang der Perforationslinien heraustrennbar. Das Heraustrennen eines Streifens aus dem Aufnahmeblatt kann mit verhältnismäßig wenig Kraft erfolgen. Der am Rand des Toilettenbeckens nach dem Abreißen verbleibende jeweilige Befestigungsbereich ist dann separat zu lösen.

Gemäß einer besonders bevorzugten Ausführungsform weist jedes Reißelement nur eine einzige Perforationslinie in dem Aufnahmeblatt auf. Beim Abreißen wird dann üblicherweise vom Nutzer nicht nur der Befestigungsbereich vom übrigen Aufnahmeblatt getrennt, sondern der Befestigungsbereich wird auch von dem Rand des Toilettenbeckens gelöst. Damit kann ein Befestigungsbereich mit einem einzigen Handgriff von der Aufnahmehilfe und von dem Rand des Toilettenbeckens zur getrennten Entsorgung von dem Aufnahmeblatt abgerissen werden.

Die vorliegende Erfindung wird nun anhand der beigefügten Zeichnungen näher erläutert, in denen zeigen:
- Fig. 1: eine erfindungsgemäße Aufnahmehilfe für WC-Toilettenbecken im Verwendungszustand in einer perspektivischen Ansicht;
- Fig. 2: die Aufnahmehilfe von Fig. 1 in einer Ansicht gemäß dem eingezeichneten Schnitt II-II in Fig. 1;
- Fig. 3: die Unterseite der Aufnahmehilfe von Fig. 1 in einem ausgefalteten Zustand mit Befestigungsbereichen;
- Fig. 4: eine vergrößerte Ansicht eines Befestigungsbereichs gemäß einer ersten Ausführungsform und
- Fig. 5: eine vergrößerte Ansicht eines Befestigungsbereichs gemäß einer zweiten Ausführungsform.

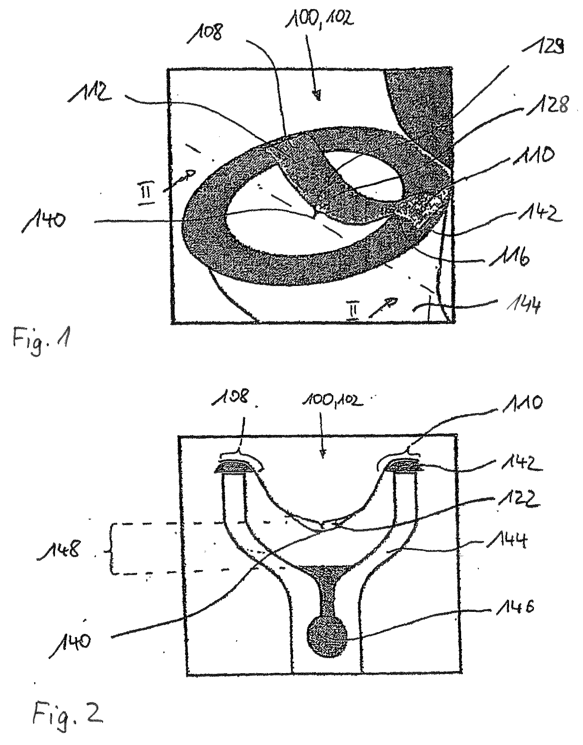

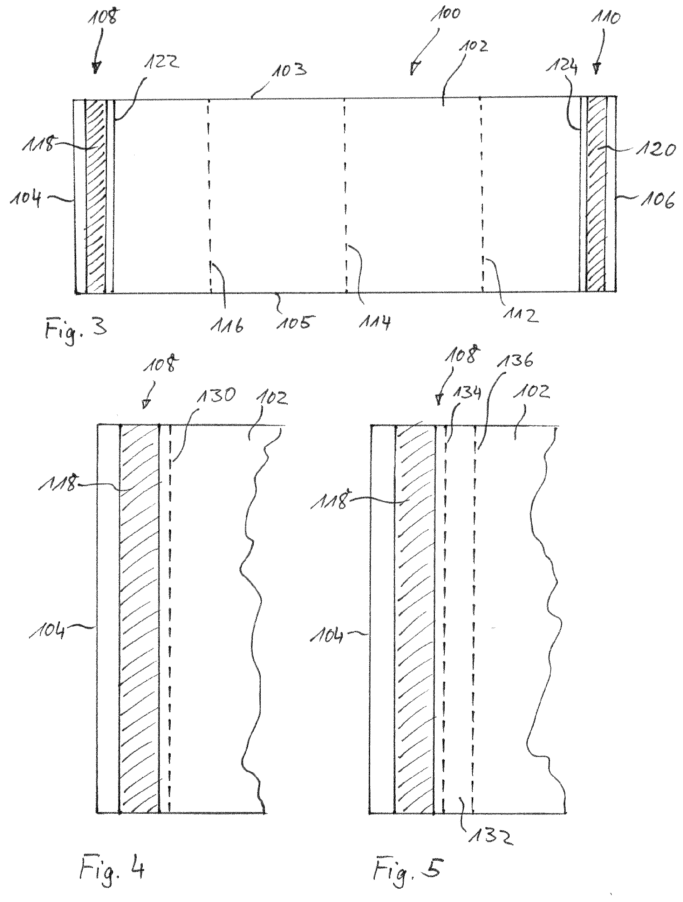

Die in den Fig. 1 bis 5 gezeigte Aufnahmehilfe 100 besteht im Wesentlichen aus einem Aufnahmeblatt 102 mit einer Länge von 40 cm bis 80 cm, vorzugsweise 48 cm. Die Breite beträgt üblicherweise zwischen 10 cm und 25 cm, vorzugsweise 15 cm. Das Aufnahmeblatt 102 ist also günstigerweise, wie dies auch in Fig. 3 dargestellt ist, rechteckförmig und besitzt zwei Breitseiten 104 und 106 sowie zwei Längsseiten 103 und 105. An der Breitseite 104 ist ein erster Befestigungsbereich 108 und an der Breitseite 106 ein zweiter Befestigungsbereich 110 vorgesehen, wobei die Breite des ersten Befestigungsbereichs 108 und des zweiten Befestigungsbereichs 110 in der Regel zwischen 1 cm und 3 cm liegt, vorzugsweise bei 2 cm. Gestrichelt eingezeichnet sind eine erste Falzstelle 112, eine zweite Falzstelle 114 und eine dritte Falzstelle 116, an denen das Aufnahmeblatt 102 zum Transport bzw. zur Lagerung gefalzt wird.

Die Befestigungsbereiche 108 und 110 bilden im vorliegenden Beispiel jeweils ein längliches Rechteck, dessen Längsachse parallel zur jeweiligen Breitseite 104 bzw. 106 verläuft. Die Befestigungsbereiche dienen dazu, die Aufnahmehilfe 100 am Rand des WC-Toilettenbeckens zu befestigen, was unten im Zusammenhang mit den Fig. 1 und 2 näher erläutert werden wird. Jeder Befestigungsbereich 108, 110 besitzt hier einen Klebebereich 118, 120. Der Klebebereich kann auf dem ansonsten weißen, günstigerweise aus Papier gefertigten Aufnahmeblatt 2 farblich abgehoben sein. Er ist damit rein optisch leicht erkennbar, auch wenn der Klebstoff an sich transparent ist.

Auf den ersten Klebebereich 118 und den zweiten Klebebereich 120 ist eine Klebeschicht aufgebracht, um die klebende Wirkung für die Befestigungsfunktion zu erzielen. Für die jeweilige Klebeschicht wird insbesondere ein Adhäsiv-Kleber verwendet, der zum einen eine ausreichende Klebekraft besitzt, um die Aufnahmehilfe während der Verwendung auf dem WC-Toilettenbecken zu halten, und andererseits nach Gebrauch von dem Toilettenbeckenrand leicht lösbar ist.

Bei dem Klebstoff handelt es sich vorzugsweise um einen Schmelzhaftkleber, der sich in einer dünnen Schicht auf das Papier aufsprühen lässt, wodurch einerseits Klebstoff bei der Herstellung eingespart wird und andererseits dünnes Papier zur Herstellung verwendet werden kann, welches ebenfalls kostengünstiger ist als dickeres Spezialpapier, welches bei anderen Beschichtungsmethoden verwendet werden müsste. Des Weiteren ist die Breite des ersten Befestigungsbereichs 108 und des zweiten Befestigungsbereichs 110 mit einer Sicherheitstoleranz beaufschlagt, so dass der erste Klebebereich 118 und der zweite Klebebereich 110 sich nicht über die gesamte Breite des jeweiligen Befestigungsbereichs erstrecken. Dies ist in Fig. 3 erkennbar, denn der jeweilige Klebebereich 118, 120 liegt zwischen dem Reißelement 122, 124 und der jeweiligen Breitseite 104, 106, aber nicht unmittelbar abgrenzend an das Reißelement bzw. die Seite.

Die Aufnahmehilfe 100 wird zweckmäßigerweise über die Toilettenspülung entsorgt. Daher besteht das Aufnahmeblatt 102 der Aufnahmehilfe 100 vorzugsweise aus einem in Wasser langsam zerfallenden Material, insbesondere Papier, und ist daher vollständig biologisch abbaubar.

Die Befestigungsbereiche 108 und 110 weisen jedoch Befestigungsmittel auf, die üblicherweise wenig gut biologisch abbaubar sind. Im einfachsten Fall handelt es sich bei den Befestigungsmitteln, wie erwähnt, um eine Klebeschicht jeweils in den Klebebereichen 118 und 120. Es kann sich aber auch um mechanische Mittel handeln, mit denen die Befestigung über Formschluss hergestellt wird, z. B. kleine Kunststoffhaken. Die mangelnde biologische Abbaubarkeit dieser Befestigungsbereiche führt dazu, dass auf dem Abwasserpfad Probleme der eingangs geschilderten Art auftreten. Trotzdem soll aber die Aufnahmehilfe im Wesentlichen über die Toilette entsorgt werden können.

Erfindungsgemäß ist daher vorgesehen, dass die Befestigungsbereiche 108, 110 von dem übrigen Aufnahmeblatt 102 definiert durch eine einfache Handbewegung ohne Zuhilfenahme von Hilfsmitteln, die nicht zu der Aufnahmehilfe gehören, abgetrennt werden können. Dazu ist ein erstes Reißelement 122 zum Abtrennen des ersten Befestigungsbereichs 108 und ein zweites Reißelement 124 zum Abtrennen des zweiten Befestigungsbereichs 110 vorgesehen. Die beiden Reißelemente 122 und 124 stellen den Rand des jeweiligen Befestigungsbereichs 108, 110 dar und erstrecken sich im Wesentlichen parallel zu den Bereitseiten 104, 106.

Bei dem Reißelement handelt es sich beispielsweise um einen Faden, der in das Papier des Aufnahmeblatts 102 integriert ist. Er ragt beispielsweise geringfügig aus einer der Längsseiten 103, 105 hervor, so dass er leicht ergriffen werden kann. Das Aufnahmeblatt 102 erstreckt sich über die Reißfäden hinweg in die Befestigungsbereiche 108 und 110 und dient gleichzeitig als Träger für die Befestigungsmittel und insbesondere die Klebeschichten auf den Klebebereichen 118 und 120.

In den Fig. 1 und 2 ist gezeigt, wie das Aufnahmeblatt 102 mit seinem ersten bzw. zweiten Befestigungsbereich 108, 110 an einem Beckenrand 142 eines WC-Toilettenbeckens 144 frei herunterhängt, ohne in Kontakt mit dem Toilettenwasser 146 zu kommen, wobei ein Sicherheitsabstand 148 verbleibt. Dadurch wird gewährleistet, dass kein Kontakt zwischen Stuhlprobe und Toilettenwasser 146 entsteht. Der Kontakt zwischen Urin und Stuhlprobe wird dadurch vermieden, dass das Aufnahmeblatt 102, wie in Fig. 2 zu sehen ist, im hinteren Bereich des Beckenrands 142 des WC-Toilettenbeckens 144 angebracht ist.

Die Aufnahmehilfe 100 kann gemäß der Druckschrift EP 1 596 721 B1 einen Aufnahmebereich 128 für den Stuhl besitzen (vgl. Fig. 1 und 2; in Fig. 3 nicht eingezeichnet), wobei der Aufnahmebereich 128 muldenförmig ausgebildet ist. Die Mulde wird durch entsprechende Verwerfungen 129 und Falzkanten 140 gebildet.

Nach der Benutzung soll nun der wesentliche Teil des Aufnahmeblatts 102 zusammen mit der Stuhlprobe jedoch ohne die Befestigungsbereiche 108 und 110 über die Toilette entsorgt werden. Dazu werden die Reißelemente 122, 124 ab- oder eingerissen, so dass diese entlang einer definierten Linie von dem übrigen Aufnahmeblatt 102 getrennt sind. Der mittlere Teil des Aufnahmeblatts 102 samt Stuhlprobe fällt dann in das Toilettenbecken, während die beiden Befestigungsbereiche 108 und 110 noch am Beckenrand kleben bzw. vom Nutzer in den Händen gehalten werden. Sie können dann separat im Hausmüll entsorgt werden.

Für den Fall, dass es sich bei den Reißelementen 122, 124 um Reißfäden handelt, können diese einfach mit einer Hand aus der Aufnahmehilfe herausgerissen werden, wobei das Aufnahmeblatt 102 gegebenenfalls mit der anderen Hand auf der bezogen auf das Reißelement 122, 124 dem jeweiligen Klebebereich 118, 120 gegenüberlegenden Seite gehalten wird. Die Befestigungsbereiche 108 und 110 bleiben dann nach dem Abtrennen an dem Beckenrand 142 kleben, während der mittlere Bereich des Aufnahmeblatts 102 in die Toilette gefallen ist. Die Befestigungsbereiche 108 und 110 werden in einem anschließenden Schritt von dem Beckenrand 142 gelöst und separat entsorgt.

Besonders bevorzugte Ausführungsformen sind in den Fig. 4 und 5 dargestellt, wobei diese Figuren jeweils eine vergrößerte Ansicht der linken Breitseite 104 darstellen. Im Fall von Fig. 4 wird das Reißelement durch eine einzige Perforationslinie 130 gebildet. Die Perforation dieser Perforationslinie 130 ist so ausgebildet, dass sie genügend Festigkeit während der Benutzung des Aufnahmeblatts bietet und andererseits der Befestigungsbereich 108 leicht von dem Aufnahmeblatt 102 definiert entlang dieser Perforationslinie 130 abgerissen werden kann. Die Perforationslinie 130 verläuft parallel zu der Breitseite 104.

Die Perforationslinie 130 ist besonders einfach in das Aufnahmeblatt 102 einzubringen, verglichen mit anderen Reißelementen. Das Aufnahmeblatt 102 erstreckt sich bis in den Befestigungsbereich 108 und letzterer wird vor dem Abreißen lediglich durch diese Perforationslinie 130 geometrisch getrennt.

Im Gebrauchszustand ist der Befestigungsbereich 108 auf die Kante des Toilettenbeckens geklebt. Zum Abreißen entlang der Perforationslinie 130 wird beispielsweise ein Finger der einen Hand des Nutzers auf einen Abschnitt rechts neben der Perforationslinie 130 gelegt. Der Finger presst dann das Aufnahmeblatt 102 gegen den Beckenrand und mit der anderen Hand kann der Benutzer den Befestigungsbereich 108 bzw. den Klebestreifen entlang der Perforationslinie 130 abreißen. In einem einzigen Arbeitsschritt wird somit der Befestigungsbereich 108 abgetrennt und von dem Toilettenbeckenrand gelöst.

Gemäß einer alternativen Ausführungsform, die in Fig. 5 dargestellt ist, wird als Reißelement ein Streifen 132 verwendet, der zwischen zwei parallelen Perforationslinien 134 und 136 verläuft. Die beiden Perforationslinien 134 und 136 erstrecken sich parallel zur Breitseite 104 des Aufnahmeblatts 102. Zum Abtrennen des Befestigungsbereichs 108 ist dann ähnlich dem Reißfaden lediglich der Streifen 132 aus dem Aufnahmeblatt 102 entlang der Perforationslinie 134, 136 herauszureißen. Der Befestigungsbereich 108 bleibt zunächst auf dem Toilettenbeckenrand kleben und muss in einem separaten Handhabungsschritt abgezogen werden.

In vorteilhafter Weise lassen sich mit den oben dargestellten Ausführungsformen von Aufnahmehilfen die Befestigungsbereiche bzw. Klebestreifen durch einfache Arbeitsschritte vom Aufnahmeblatt trennen und sachgerecht entsorgen. Damit sind weniger Probleme beim Abfluss in Kanalsystemen zu erwarten, und es kann den Umweltvorlagen leichter Genüge getan werden.

## Patentansprüche

1. Aufnahmehilfe (100) für WC-Toilettenbecken zur Aufnahme von Stuhlproben mit
- einem Aufnahmeblatt (102), das langgestreckt ist und dessen Länge ein Vielfaches seiner Breite beträgt, wobei
- das Aufnahmeblatt (102) einen ersten und mindestens einen zweiten Befestigungsbereich (108, 110) zur lösbaren Befestigung im Bereich eines Beckenrandes (142) eines WC-Toilettenbeckens (144) aufweist, **dadurch gekennzeichnet, dass**
- die Befestigungsbereiche (108, 110) jeweils durch mindestens ein Reißelement (122, 124) von dem übrigen Aufnahmeblatt (102) geometrisch getrennt sind,
- und das mindestens eine Reißelement (122, 124) ein definiertes Abreißen des jeweiligen Befestigungsbereichs (108, 110) vom übrigen Aufnahmeblatt (102) gewährleistet,
- das Aufnahmeblatt (102) rechteckförmig ausgebildet ist und an jeder Breitseite (104, 106) jeweils einer der Befestigungsbereiche (108, 110) angeordnet ist, und
- parallel zu jeder Breitseite (104, 106) jeweils das Reißelement (122, 124) zum Abreißen des jeweiligen Befestigungsbereichs verläuft, das sich über die gesamte Breite des Aufnahmeblatts erstreckt.

2. Aufnahmehilfe nach Anspruch 1, wobei das Aufnahmeblatt (102) einteilig mit den Befestigungsbereichen (108, 110) gebildet ist.

3. Aufnahmehilfe nach einem der vorhergehenden Ansprüche, wobei jeder Befestigungsbereich (108, 110) einen Klebstoff aufweist, der das lösbare Befestigen des Befestigungsbereichs an dem Beckenrand (142) des WC-Toilettenbeckens (144) gewährleistet.

4. Aufnahmehilfe nach Anspruch 3, wobei der Klebstoff ein aufgesprühter Schmelzhaftkleber ist.

5. Aufnahmehilfe nach einem der vorhergehenden Ansprüche, wobei jedes Reißelement (122, 124) eine Perforationslinie (130, 136, 136) in dem Aufnahmeblatt (102) aufweist.

6. Aufnahmehilfe nach einem der vorhergehenden Ansprüche, wobei jedes Reißelement (122, 124) zwei parallele Perforationslinien (134, 136) aufweist, und ein Streifen (132) des Aufnahmeblatts (102) zwischen den parallelen Perforationslinien zum Abreißen des jeweiligen Befestigungsbereichs (108, 110) vom übrigen Aufnahmeblatt entlang der Perforationslinien heraustrennbar ist.

## Claims

1. Receiving aid (100) for WC toilet bowls for receiving fecal specimens including
- a receiving sheet (102), which is elongated and the length of which is a multiple of its width, wherein
- the receiving sheet (102) has a first and at least a second fixing area (108, 110) for detachably fixing in the area of a bowl edge (142) of a WC toilet bowl (144),
**characterized in that**
- the fixing areas (108, 110) are each geometrically separated from the remaining receiving sheet (102) by at least one tear element (122, 124),
- and the at least one tear element (122, 124) ensures defined tearing of the respective fixing area (108, 110) from the remaining receiving sheet (102),
- the receiving sheet (102) is rectangularly formed and each one of the fixing areas (108, 110) is disposed at each short end (104, 106), and
- parallel to each short end (104, 106), the tear element (122, 124) for tearing the respective fixing area respectively extends, which extends across the entire width of the receiving sheet.

2. Receiving aid according to claim 1, wherein the receiving sheet (102) is formed integrally with the fixing areas (108, 110).

3. Receiving aid according to any one of the preceding claims, wherein each fixing area (108, 110) has an adhesive ensuring the detachable fixation of the fixing area to the bowl edge (142) of the WC toilet bowl (144).

4. Receiving aid according to claim 3, wherein the adhesive is a sprayed-on melting contact adhesive.

5. Receiving aid according to any one of the preceding claims, wherein each tear element (122, 124) has a perforation line (130, 136, 136) in the receiving sheet (102).

6. Receiving aid according to any one of the preceding claims, wherein each tear element (122, 124) has two parallel perforation lines (134, 136), and a strip (132) of the receiving sheet (102) is separable along the perforation lines from the remaining receiving sheet between the parallel perforation lines for tearing the respective fixing area (108, 110).

## Revendications

1. Aide à la réception (100) pour cuvettes de toilettes de WC, destinée à recueillir des échantillons de selles, avec
- une feuille de réception (102), qui est allongée et dont la longueur est un multiple de sa largeur,
- la feuille de réception (102) présentant une première et au moins une deuxième zone de fixation (108, 110) pour la fixation amovible dans le secteur d'un bord de cuvette (142) d'une cuvette de toilettes de WC (144), **caractérisée en ce que**
- les zones de fixation (108, 110) sont séparées géométriquement respectivement par au moins un élément à déchirure (122, 124) du reste de la feuille de réception (102)
- et le au moins un élément à déchirure (122, 124) garantit un arrachage déterminé de la zone de fixation (108, 110) respective du reste de la feuille de réception (102),
- la feuille de réception (102) est constituée en forme de rectangle et est agencée sur chaque côté large (104, 106) respectivement de l'une des zones de fixation (108, 110) et
- parallèlement à chaque côté large (104, 106), respectivement, l'élément à déchirure (122, 124) évolue pour arracher la zone de fixation respective, qui s'étend sur toute la largeur de la feuille de réception.

2. Aide à la réception selon la revendication 1, la feuille de réception (102) étant formée d'une seule pièce avec les zones de fixation (108, 110).

3. Aide à la réception selon l'une des revendications précédentes, chaque zone de fixation (108, 110) présentant un adhésif, qui garantit la fixation amovible de la zone de fixation sur le bord (142) de la cuvette (144) des toilettes de WC.

4. Aide à la réception selon la revendication 3, l'adhésif étant un adhésif thermofusible appliqué par pulvérisation.

5. Aide à la réception selon l'une des revendications précédentes, chaque élément à déchirure (122, 124) présentant une ligne de perforations (130, 136, 136) dans la feuille de réception (102).

6. Aide à la réception selon l'une des revendications précédentes, chaque élément à déchirure (122, 124) présentant deux lignes de perforations parallèles (134, 136) et une bande (132) de la feuille de réception (102) entre les lignes de perforations parallèles est séparable, pour arracher la zone de fixation (108, 110) respective du reste de la feuille de réception le long des lignes de perforations.
